# EUROPEAN PATENT SPECIFICATION

(11) **EP 1 776 453 B1**
(45) Date of publication and mention of the grant of the patent: **25.02.2009**
(21) Application number: 05785460.6
(22) Date of filing: 11.08.2005
(51) Int. Cl.: C12N 5/06

(54) **ISOLATION OF ENDOTHELIAL PROGENITOR CELL SUBSETS AND METHODS FOR THEIR USE**
ISOLATION DER ENDOTHELVORLÄUFERZELLEN-UNTERMENGEN UND VERFAHREN ZU IHRER VERWENDUNG
ISOLEMENT DE SOUS-ENSEMBLES DE CELLULES PROGENITRICES ENDOTHELIALES ET METHODES D'UTILISATION DE CES SOUS-ENSEMBLES

(30) Priority: 13.08.2004 US 601188 P
(43) Date of publication of application: 25.04.2007
(73) Proprietor: MEDTRONIC, INC., Minneapolis MN 55432-5604 (US)
(72) Inventor: POPA, Eliane, R., NL-9737 PH Groningen (NL); HARMSEN, Martin, C., NL-9765 CH Paterswolde (NL); VAN DER STRATE, Barry, W.A., NL-9745 DN Groningen (NL); VAN LUYN, Marja, J.A., NL-9721 WX Groningen (NL); HENDRICKS, Marc, NL-6441 KD Brunssum (NL)
(74) Representative: Green, Mark Charles
(86) International application number: PCT/US2005/028923
(87) International publication number: WO 2006/020954

(56) References cited:
- EP-A- 1 270 719
- WO-A-93/06792
- WO-A-97/18842
- WO-A-02/097066
- WO-A-20/04098499
- ALESSANDRI G ET AL: "Human vasculogenesis ex vivo: Embryonal aorta as a tool for isolation of endothelial cell progenitors" LABORATORY INVESTIGATION, UNITED STATES AND CANADIAN ACADEMY OF PATHOLOGY, BALTIMORE,, US, vol. 81, no. 6, June 2001 (2001-06), pages 875-885, XP002227166 ISSN: 0023-6837
- PEICHEV MM ET AL: "Expression of VEGFR-2 and AC133 by circulating human CD34+ cells identifies a population of functional endothelial precursors" BLOOD, W.B.SAUNDERS COMPANY, ORLANDO, FL, US, vol. 95, no. 3, 1 February 2000 (2000-02-01), pages 952-958, XP002251073 ISSN: 0006-4971
- MANCUSO P ET AL: "Resting and activated endothelial cells are increased in the peripheral blood of cancer patients" BLOOD, W.B.SAUNDERS COMPANY, ORLANDO, FL, US, vol. 97, no. 11, 1 June 2001 (2001-06-01), pages 3658-3661, XP002274898 ISSN: 0006-4971
- MASUDA H ET AL: "ENDOTHELIAL PROGENITOR CELLS FOR REGENERATION" HUMAN CELL - HITO SAIBO KENKYUKAI ZASSHI, TOKYO, JP, vol. 13, no. 4, December 2000 (2000-12), pages 153-160, XP008012546 ISSN: 0914-7470

## Description

### FIELD OF THE INVENTION

The present description of the invention relates generally to methods of isolating endothelial progenitor cells for the treatment of cardiovascular disease, description wherein the progenitor cells of the invention and the progenitor cells of the therapeutic composition of the invention are not of human embryonic origin, and wherein the use of human embryos for commercial or industrial purposes is excluded from the method for isolation of endothelial progenitor cells of the invention.

### BACKGROUND OF THE INVENTION

The development of new blood vessels in response to tissue ischemia constitutes a natural host reaction intended to maintain tissue perfusion required for physiologic organ function. This natural angiogenesis is impaired in advanced age, diabetes and hypercholesterolemia. In each of these conditions, there is a reduction in endogenous expression of vascular endothelial growth factor (VEGF) and exogenous VEGF administration leads to enhanced neovascularization.

Ischemic tissue injury triggers a series of events, including mobilization and recruitment of circulating progenitor cells (CPC) to the injury site. In models of post-ischemic angiogenesis, a subpopulation of CPC, namely endothelial progenitor cells (EPC), incorporate into neovessels. Moreover, in animal models, as well as in clinical settings of acute myocardial infarction (aMI), systemic administration of EPC contributes to revascularization of the myocardium and is associated with improved myocardial function.

Since their original description, bone marrow-derived EPC have become a focal point in regenerative therapy following evolving vascular damage. Because numbers of CPC, which are normally low in peripheral blood, increase significantly after an ischemic event, a causal link between vascular damage and CPC-mediated repair has been postulated. In animal models of angiogenesis following ischemia, bone marrow-derived EPC incorporate into neovessels. Moreover, local and systemic levels of angiogenic growth factors, including VEGF, rise after ischemia and are associated with increased numbers of circulating CPC.

The obvious therapeutic potential of exogenous growth factor administration has been successfully assessed in animals and humans. In various animal models, mobilization of EPC after vascular damage by administration of VEGF, granulocyte macrophage colony stimulating factor (GM-CSF), granulocyte colony stimulating factor (G-CSF), fibroblast growth factor 1 (FGF-1), stromal derived factor 1 (SDF-1) or a statin drug, positively correlated with increased numbers of circulating EPC and improved therapeutic neovascularization. Direct evidence for the vasculogenic potential of EPC has been provided by studies in which EPC transplanted in mice with hind limb ischemia incorporated into newly formed blood vessels (Kalka C. et al., "Transplantation of ex vivo expanded endothelial progenitor cells for therapeutic neovascularization," Proc. Natl. Acad. Sci. 97:3422-7, 2000). In a murine model of myocardial infarction (Ml) intravenous injection of human CD34⁺ CPC contributed to revascularization of the myocardium, and was associated with salvage of myocardial function (Kocher A.A. et al., "Neovascularization of ischemic myocardium by human bone marrow-derived angioblasts prevents cardiomyocyte apoptosis, reduces remodeling and improves cardiac function," Nat. Med. 7:412-3, 2001). Moreover, intracoronary infusion of autologous EPC into the infarct artery in patients with aMI resulted in increased myocardial viability in the infarct area (Assmus B. et al., "Transplantation of progenitor cells and regeneration enhancement in acute myocardial infarction [TOPCARE-AMI]," Circulation 106:3009-17, 2002).

Current progenitor cell research is focused on the clinical application of CPC in therapeutic neovascularization. Therefore, future large-scale therapeutic application of CPC will require an understanding of the phenotypic and functional properties of these cells. It has been demonstrated that phenotypically diverging subsets of CPC can be distinguished. The cell surface markers CD34, CD133 and VEGFR-2 (KDR, flk-1) have been used as CPC markers for single- and dual-parameter flow cytometric analysis of CPC, which leads to enrichment of CD34⁺ progenitor cells (PC). The shortcomings of this approach are technical limitations and include restrictions in determining the identity and relationship between CPC subsets when they are defined by single and dual parameter detection.

Interestingly, the methods used for CPC detection and isolation also determine the outcome of CPC functional assays. When isolated by flow cytometry and cultured under angiogenic conditions, CD34⁺ CPC form spindle-shaped cells, which, over time, organize in capillary-like structures. Moreover, these cells express markers specific for mature endothelial cells (EC) such as CD31, E-selectin and Tie-2.

Alternatively CPC have been isolated based on *in vitro* culture of mononuclear cells on fibronectin- or gelatin-coated plates in the presence of angiogenic growth factors. Isolated adherent cells that were low density lipoprotein (LDL) negative and exhibited lectin-binding ability were called CPC. Although these cells promote angiogenesis *in vivo,* they have monocytic features and their angiogenicity is actually caused by their production of angiogenic factors, such as VEGF, hepatocyte growth factor (HGF), G-CSF and GM-CSF.
Thus, while these LDL ⁻, lectin-binding cells do not directly form EC, they can modulate angiogenesis.

EP 1270719 (Viacell) and WO02/097066 (Medestea Intemazionales et al) both disclose EPC's which are lineage negative and which express CD34 on their surface. These documents also provide a method for the isolation of lineage negative EPC's and their sorting into specific subpopulations by antibody-coated-magnetic-bead selection.

Mancuso, in "Blood" pub. W.B. Saunders & Co, Orlando, FL, USA, vol 97, no. 11, 1 June 2001, pages 3658-3661 discloses the use of a 4-colour flow cytometry procedure for the evaluation of circulating endothelial cells.

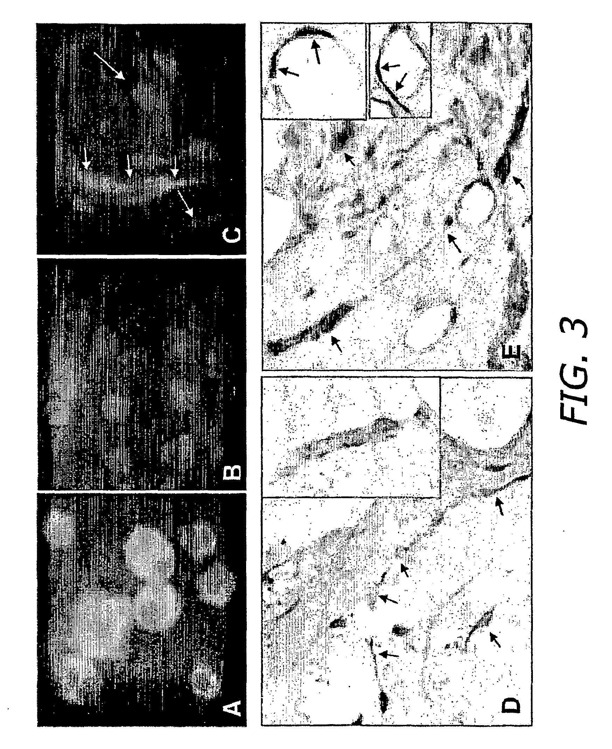

Masuda, in "Endothelial Progenitor Cells for Regeneration", Human Cell-Hito Saibo Kenkyukai Zasshi, Tokyo, JP, vol. 13 no. 4, December 200, pages 153-160 discloses that enrichment of EPC in vivo is achieved by cell sorting (Fig.3).

WO93/06792 (SciMed) and WO97/18842 (Fidia Advanced Biopolymers at al) disclose matrices for cell growth.

Based on the foregoing, both heterogenous and homogenous populations of endothelial progenitor cells present an opportunity for treatment of cardiovascular disease. Therefore, methods for sorting and isolating specific populations of cells suitable for use in regenerative therapy are needed.

### SUMMARY OF THE INVENTION

The present description discloses methods for the isolation of human peripheral blood endothelial progenitor cells yielding cells which can form blood vessels or induce angiogenesis and inflammation-mediating cells using four-parameter fluorescence activated cell sorting.

In one embodiment of the present invention a method is provided for isolation of endothelial progenitor cells according to claim 1.

The antibodies useful for identifying lineage-committed cells include antibodies specific for the cell markers CD3, CD14, CD16/56, CD19 and CD31.

In another embodiment of the present invention, the reacting step comprises reacting the lineage-negative cells with antibodies specific for the cell markers CD34, CD133 and KDR. In another embodiment of the present invention, the resulting endothelial progenitor cells express CD34. In yet another embodiment of the present invention the resulting endothelial progenitor cells are CD34⁺CD133⁻KDR⁻.

In one embodiment of the present invention, the endothelial progenitor cells are blood vessel generating cells, inflammation-mediating cells or both. In another embodiment of the present invention, the blood vessel-generating cells are CD34⁺CD133⁻KDR⁻ endothelial progenitor cells.

In another embodiment of the present invention, the endothelial progenitor cells induce angiogenic responses in surrounding blood vessels.

In one embodiment of the present invention, a therapeutic composition for inducing angiogenesis at a treatment site is provided according to claim 10.

In another embodiment of the present invention, a therapeutic composition having a chemotactic effect on inflammation-mediating cells at a treatment site is provided according to claim 13. In another embodiment of the present invention, the biodegradable biocompatible matrix contains CD34⁺CD133⁻KDR⁻ endothelial progenitor cells.

In yet another embodiment of the present invention, the biodegradable biocompatible matrix is selected from the group consisting of solubilized basement membrane, autologous platelet gel, collagen gels or collagenous substrates based on elastin, fibronectin, laminin, extracellular matrix and fibrillar proteins.

In one embodiment of the present invention an isolated population of endothelial progenitor cells is provided according to claim 16.

In an embodiment of the present invention, the isolated population of endothelial progenitor cells comprises blood vessel-generating cells. In another embodiment of the present invention, the isolated population of endothelial progenitor cells comprises inflammation-mediating cells. In another embodiment of the present invention, the isolated population of endothelial progenitor cells induce angiogenic responses in surrounding blood vessels.

### BRIEF DESCRIPTION OF THE FIGURES

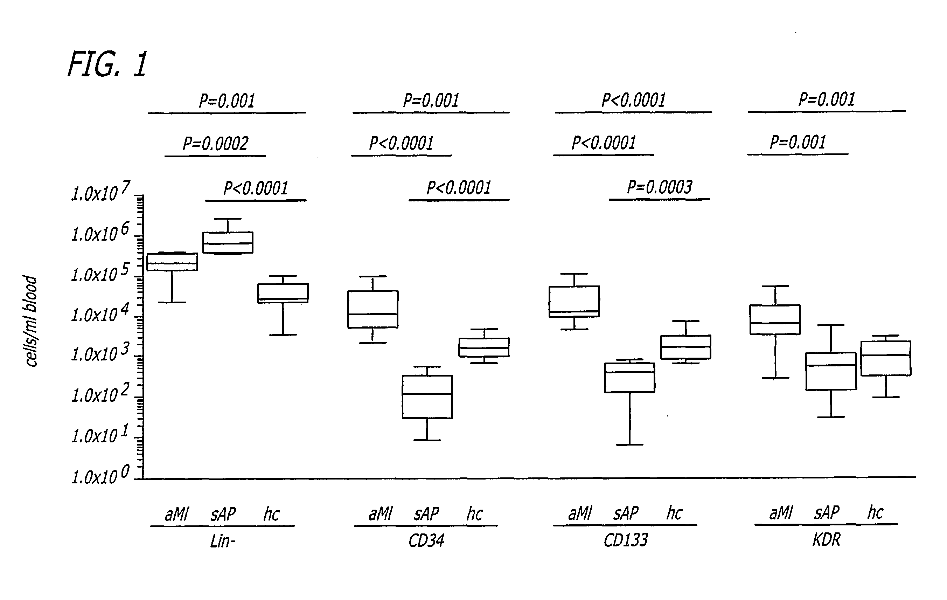

FIG. **1** depicts the numbers of circulating progenitor cells (CPC) subsets, determined by flow cytometry, in peripheral blood from patients with acute myocardial infarct (aMl, n=10), stable angina pectoris (sAP, n=10) and healthy controls (hc, n=9) defined as Lin-, CD34, CD133 or KDR according to the teachings of the present invention.

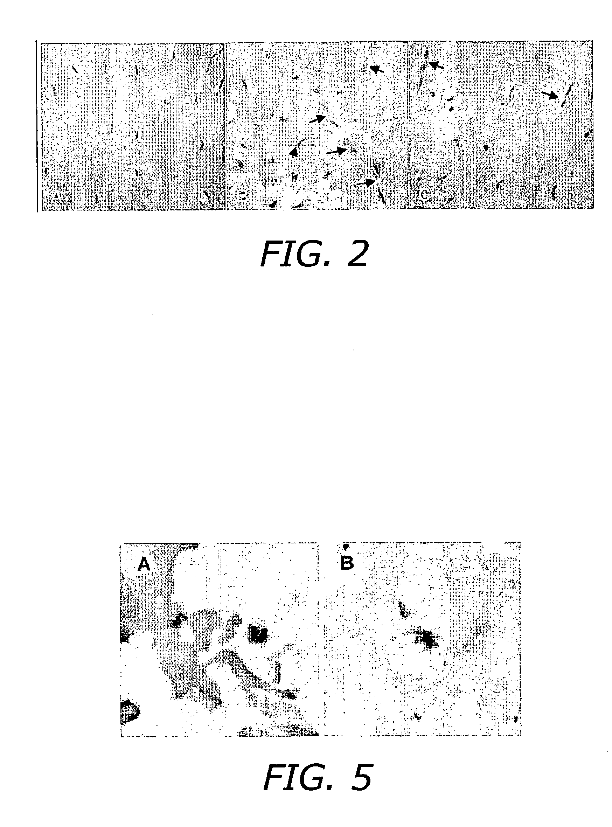

FIG. **2** depicts the *in vivo* behavior of human CPC subsets, either CD34⁺ or CD133⁺ sorted in Matrigel^{®} pellets implanted in nude mice after 14 days according to the teachings of the present invention. (A) bare Matrigel^{®}; (B) Matrigel^{®} containing CD34⁺ cells, (C) Matrigel^{®} containing CD133⁺ cells. Arrows indicate representative network structures formed by spindle-shaped cells, considered potential EPC. Objective magnification 40x.

FIG. **3** depicts morphological detection of human endothelial progenitor markers on isolated CPC enclosed in Matrigel^{®} pellets implanted in nude mice according to the teachings of the present invention. In panels A-C human endothelial cells (EC) were detected with lectin *Ulex europeus-1* agglutinin (UEA-1 conjugated to TRITC), a human- and EC-specific lectin; (A) human umbilical vein endothelial cell (HUVEC) positive control; (B) murine EC cell line (H5V) negative control; (C) cells in Matrigel^{®} seeded with human CD34⁺ EPC bound UEA-1 lectin. UEA-1-binding cells were spindle shaped and made contacts (arrows). In panels D-E the EC phenotype was confirmed by (D) staining for human CD34 (EPC, EC), and (E) CD31 (EC). Positive endothelial cells are indicated by arrows. The inserts show additional examples of human blood vessels. Objective magnification 40x.

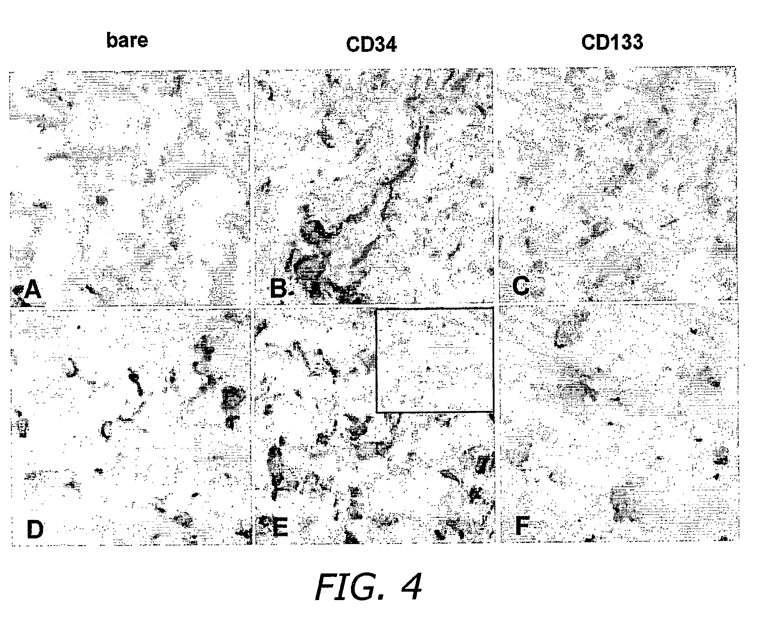

FIG. **4** depicts the murine angiogenic and inflammatory response to human CPC subsets, implanted in nude mice in Matrigel^{®}, isolated according to the teachings of the present invention. The formation of murine blood vessels in Matrigel^{®} were detected using monoclonal antibodies specific for murine CD31 (A-C). Murine monocytes/macrophages were detected with monoclonal antibodies specific to those cells (D-F). The insert in panel E shows the lack of human CD68⁺ macrophages in the section. Objective magnification 40x.

FIG. **5** depicts detection of interleukin-8 (IL-8) in CD34⁺ cells, isolated according to the teachings of the present invention, implanted in nude mice in Matrigel^{®}. Interleukin-8 expression was determined immediately after sorting (A) and 14 days after implantation (B). Objective magnification 40x.

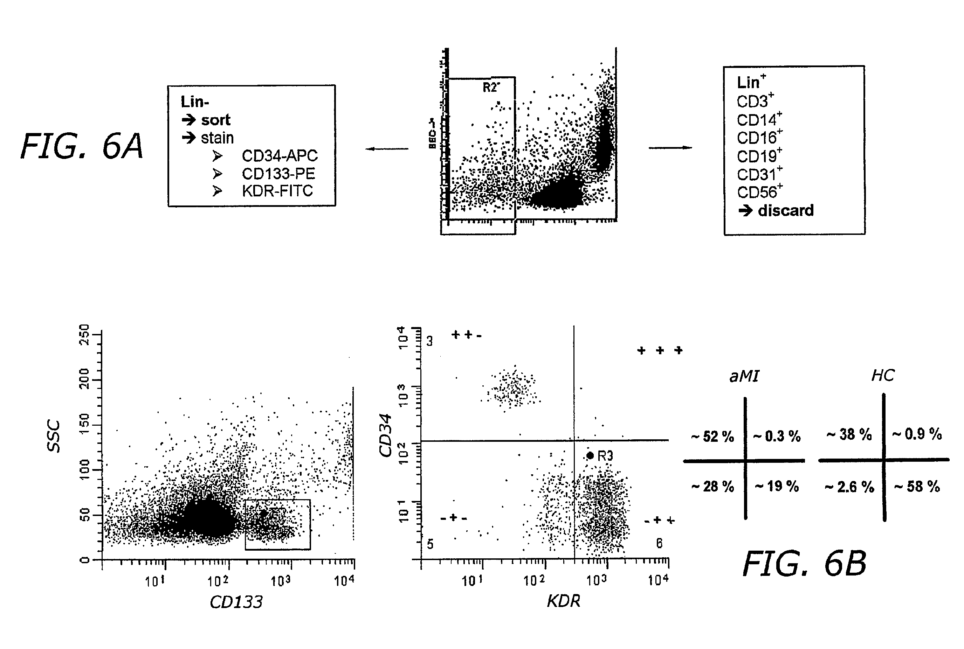

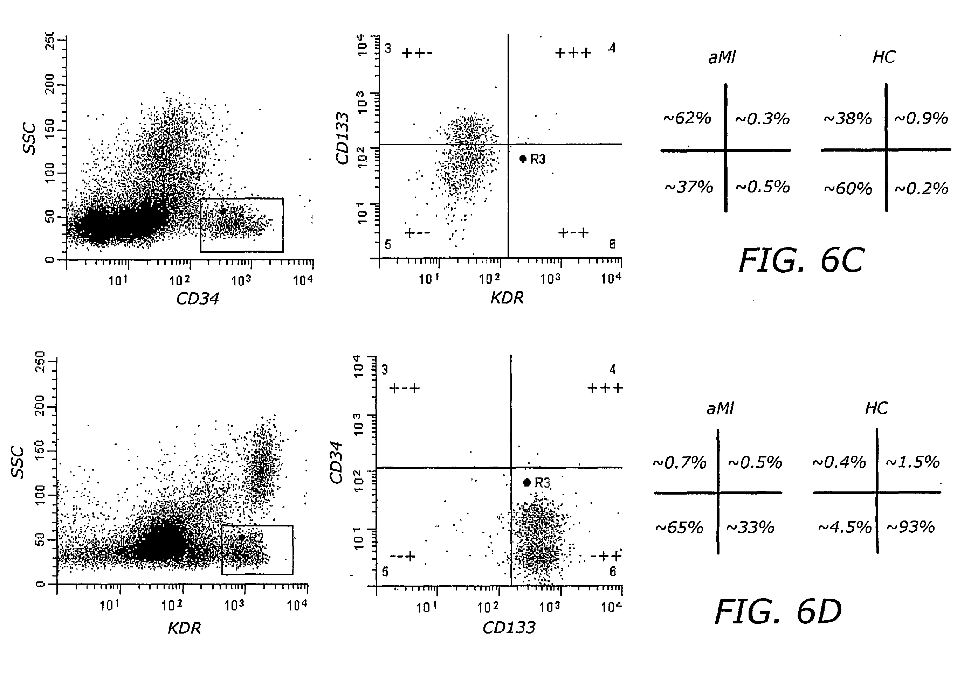

FIG. **6** depicts flow cytometric analysis of CPC subsets in peripheral blood from patients with aMl (n=10) and healthy controls (hc, n=9) according to the teachings of the present invention. The lineage-negative (Lin⁻) (A) cell population were stained with antibodies to CD34, CD133 and KDR and cells expressing each of the three markers were gated and analyzed for the expression of the remaining two markers. (B) CD133 analysis; (C) CD34 analysis; (D) KDR analysis.

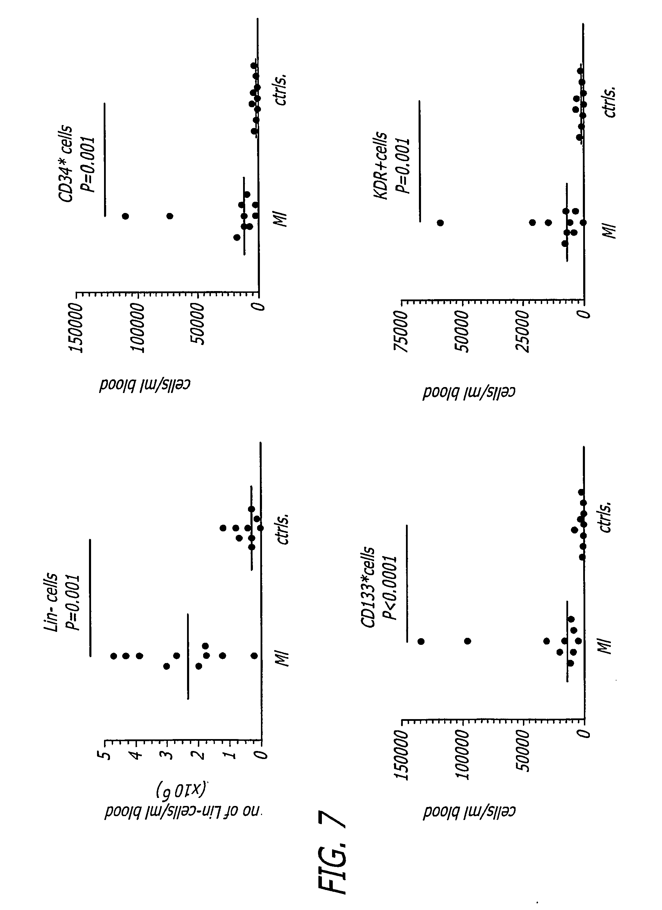

FIG. **7** depicts the number of CPC in peripheral blood from aMl patients (n=10) and healthy controls (hc, n=9) as subsets defined as Lin-, CD34⁺, CD133⁺ or KDR⁺. Dots represent individuals; horizontal lines represent the median; fold increase based on means. P=p value.

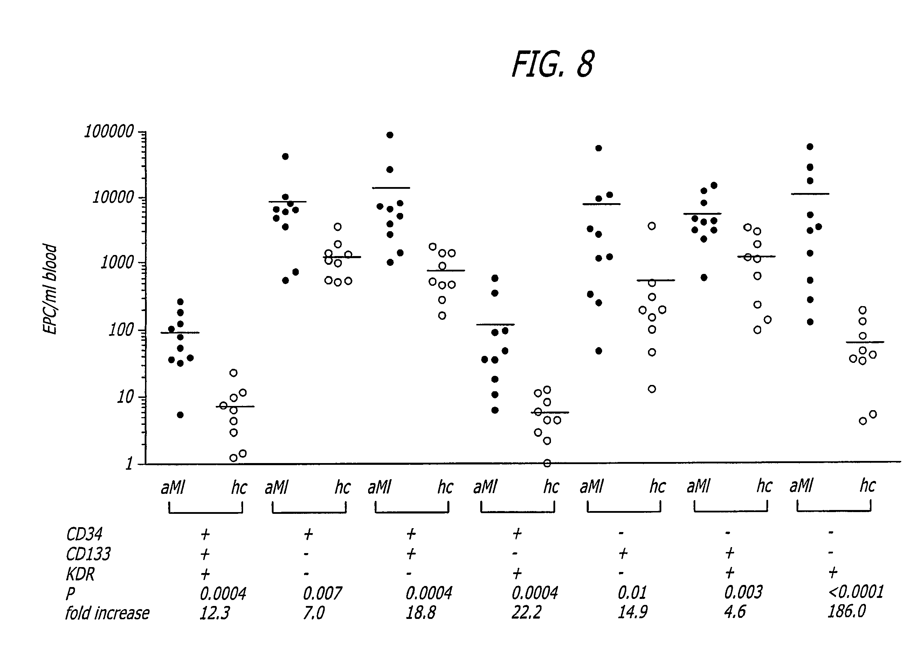

FIG. **8** depicts cell numbers, determined by flow cytometric analysis, of the seven CPC subsets defined according to the teachings of the present invention in aMl patients (n=10) and healthy controls (hc, n=9). Horizontal lines represent the median; fold increase based on means.

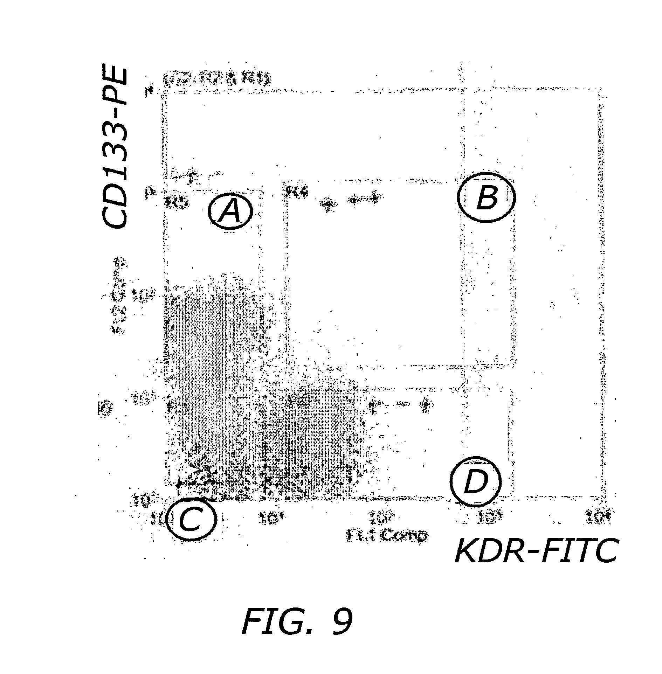

FIG. **9** depicts flow cytometric sorting of four CD34⁺ CPC subsets, or combinations of subsets according to the teachings of the present invention. (A) CD34⁺CD133⁺KDR⁻; (B) CD34⁺CD133⁺KDR⁺; (C) CD34⁺CD133⁻KDR⁻; (D) CD34⁺CD133⁻ KDR⁺.

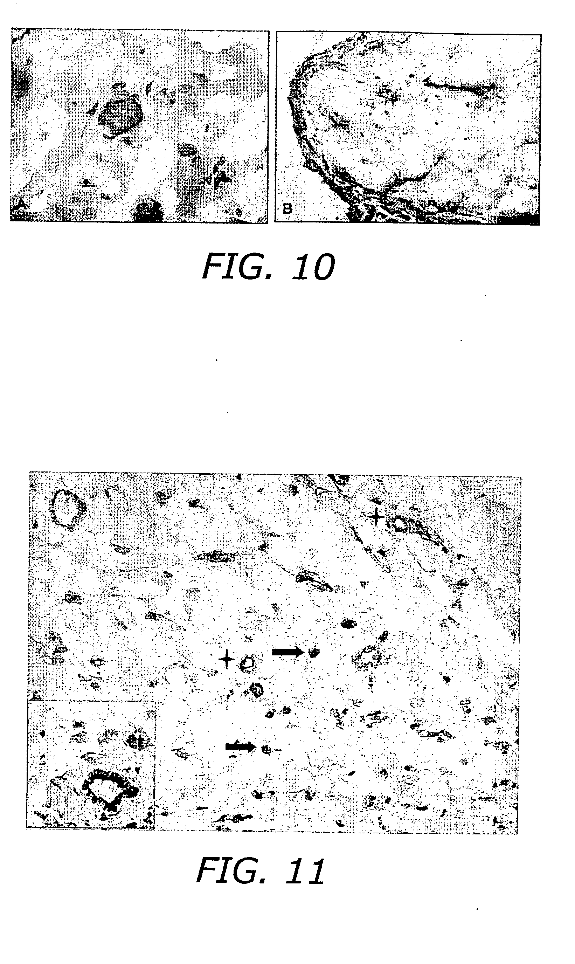

FIG. **10** depicts the presence of human CD31-positive cells in CD34⁺ CPC-loaded Matrigel^{®}, according to the teachings of the present invention, 14 days after implantation in nude mice. (A) clusters of human CD31-positive cells with an immature phenotype; (B) Human CD31-positive cells in vessel-like structures.

FIG. **11** depicts the presence of murine CD31-positive vasculature in CD34⁺CD133⁻KDR⁻ CPC-loaded Matrigel^{®}, according to the teachings of the present invention, 14 days after implantation in nude mice. Vessels of different sizes ranging from capillaries (arrows) to small (asterisk) and large (inset) vessels were detected. Both images (large and inset) were taken at the same magnification of the same section of the Matrigel^{®} pellet.

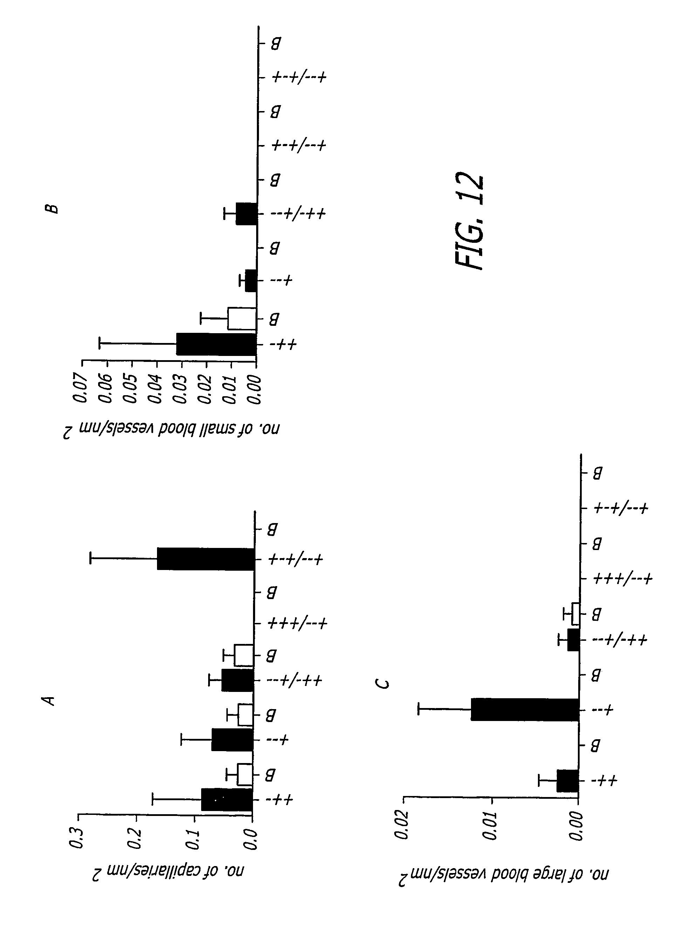

FIG. **12** depicts the quantification of murine CD31-positive blood vessels/nm² in Matrigel^{®} loaded with CD34⁺ CPC from four CD34⁺ subsets according to the teachings of the present invention, 14 days after implantation in nude mice. (A) capillaries, (B) small blood vessels and (C) large blood vessels. ++-, CD34⁺CD133⁺KDR⁻; +--, CD34⁺CD133⁻KDR⁻; +++, CD34⁺CD133⁺KDR⁺; +-+, CD34⁺CD133⁻KDR⁺; B, bare Matrigel^{®}.

### DETAILED DESCRIPTION OF THE INVENTION

Over the past several years CPC have become a focal point in cardiovascular regenerative therapy, especially since therapeutic mobilization of CPC by growth factor administration and transplantation of these cells into the infarcted region have proven beneficial for patients with ischemic conditions. Previously, a subset of CPCs, endothelial progenitor cells (EPC), have been designated as key players in neovascularization. However, there is accumulating evidence that EPC are phenotypically and functionally a heterogeneous population with endothelium-forming capacity. This heterogeneous population of CPC therefore provides a source of EPCs with different functionalities. For the purposes of describing the invention in this specification, circulating progenitor cells and endothelial progenitor cells refer to the same cell population.

The present inventors have unexpectedly discovered a subset of CPCs, which are lineage-negative and express CD34, but not CD133 and KDR, which are responsible for forming blood vessels. The CD designation refers to a "cluster of differentiation" antigen which systematically identifies antigens present on leukocyte cell surfaces. CD34 is a transmembrane glycoprotein constitutively expressed on endothelial cells and on hematopoietic stem cells. CD133 is a hematopoietic stem cell antigen also known as prominin. KDR is the precursor to the human vascular endothelial growth factor receptor 2 (VEGFR2) and is also known as Flk-1. It had been previously thought that this blood-vessel forming population of CPCs was KDR⁺.

Stem and progenitor cells lack certain markers that are characteristic of more mature, lineage-committed (Lin⁺) cells. Lineage-specific markers include, but are not limited to, CD3, CD8, CD10, CD14, CD16/56, CD19, CD20, CD31 and CD33. Lin⁺ cells express CD3, CD14, CD16/56, CD19 and CD31. Lin⁻ cells do not express CD3, CD14, CD16/56, CD19 and CD31.

The *in vivo* behavior of human CPC expressing the widely accepted CPC markers CD34, CD133 and KDR was studied by transcription profiling and fine dissection of EPC phenotypes based on the expression pattern of these markers. CPCs from three groups of patients were studied: (i) acute myocardial infarct (aMl) patients who had undergone successful reperfusion therapy, (ii) healthy volunteers and (iii) patients with stable angina undergoing treatment with statin drugs. Statin therapy has been reported to increase the levels of CPCs as early as 7 days after the initiation of treatment and many aMl patients are on statin therapy.

Peripheral blood mononuclear cells from each group of patients, sorted for the lineage-negative population and expressing either of the CPC markers CD34, CD133 or KDR, can be subdivided into a total of seven discrete subsets based on a three-parameter assessment (three-color fluorescence activated cell sorting) of cell expression of CD34, CD133 and KDR. These seven subsets were present in the circulation of healthy subjects and in stable angina patients undergoing treatment with statin drugs and were all increased in cell number after aMl.

The mobilization of all three CPC subsets (i.e. CD34⁺, CD133⁺ and KDR⁺) after aMl indicates non-preferential recruitment of progenitor cells (PC) (FIG. **6A**). Moreover, expression analysis of genes involved in endothelial cell differentiation and function revealed no major differences in gene expression within CPC of the same subset between aMl patients and healthy controls. These findings suggest that increased mobilization of CPC after aMI is not a consequence of altered expression makeup of these cells, but rather of external factors enhancing CPC detachment from the bone marrow. Additionally, vascular endothelial growth factor (VEGF), produced in the ischemic lesion, induces expression of matrix metalloproteinase-9 in the bone marrow. This process results in release of soluble Kit ligand, which drives the mobilization of cKit⁺ stem and progenitor cells to the circulation.

To study the behavior of these three CPC subsets *in vivo,* the present inventors established a model in which human CPC, enclosed in a biodegradable matrix such as Matrigel^{®} (BD Biosciences), are allowed to mature in a murine host. Matrigel^{®} is a biodegradable and biocompatible solubilized basement membrane matrix. Other biodegradable matrices as are known to those persons skilled in the art can be used within the scope of the present invention. Other examples of biodegradable matrices include, but are not limited to, autologous platelet gel, collagen gels or collagenous substrates based on elastin, fibronectin, laminin, extracellular matrix and fibrillar proteins. The use of a biodegradable matrix has a number of advantages, one of which is local confinement of CPC, which makes it possible to observe relatively low numbers of target cells. Additionally, soluble factors produced by CPC can freely diffuse through the biodegradable matrix and reach the host environment. Therefore, not only can differentiation of human CPC be monitored, but the impact of these cells on host-derived angiogenesis (i.e. sprouting of surrounding murine blood vessels) is readily visible. Additionally, biodegradable matrices are useful in the implantation of CPC into mammals for the treatment of diseases that will benefit from the localized transplantation of progenitor cells. Finally, the interplay between human CPC and murine inflammatory cells can be studied, thus providing indications regarding the role of EPC in inflammatory remodeling after ischemia.

CD34⁺ cells, encapsulated in Matrigel^{®} implants, but not CD133⁺ cells or KDR⁺ cells, formed mature endothelial cells (EC) (FIG. **2B**). Gene expression analysis revealed that CD31 transcripts were present in all three subsets, suggesting endothelium-forming potential. CD31 has served as a surrogate for endothelial cells with a monocytic phenotype, or monocytes with angiogenic potential, whereby the presence of CD31 transcripts as a proof for endothelial commitment is attenuated. Von Willebrand Factor transcripts were detectable in CD34⁺ cells and CD133⁺ cells, but not always in KDR⁺ cells, indicating that the differentiation of the former two subsets may indeed be skewed towards the endothelial lineage.

The CD34⁺ population which gives rise to mature endothelial cells also expresses Tie-2, a tyrosine kinase receptor. Gene transcripts for CD34, Tie-1, Tie-2, VEGF and KDR, which are characteristic of CPC, were present in CD34⁺ cells and at low levels in CD133⁺ cells but were absent in KDR⁺ cells, indicating a stronger commitment of the former two subsets to the endothelial lineage. Tie-2 expression was found only in the CD34⁺ subset, which was the only subset giving rise to endothelial cells *in vivo.* Since Tie-2 is essential for endothelial cell survival and capillary morphogenesis, the presence of this molecule may be instrumental for endothelial cell formation in this subset *in vivo.*

In order to distinguish discrete, phenotypically distinct CPC subsets of lineage-negative cells, three-parameter flow cytometry analysis of the CPC markers CD34, CD133 and KDR was established. The advantage of this strategy, as compared to previous techniques, consists of an unbiased inclusion of all CPC subtypes in the analysis. This unbiased inclusion is accomplished by the use of uncommitted progenitor cells, enriched for lineage-negative (Lin⁻) cells rather than CD34 pre-selection, as the starting population for analysis. Because this approach allows the dissection of CPC populations implanted *in vivo* (i.e. CD34⁺, CD133⁺, KDR⁺ cells), probable CPC subsets responsible for the observed *in vivo* effects are identified. Using this technique, seven phenotypically distinct CPC subsets within the major CD34⁺, CD133⁺ and KDR⁺ cell populations were identified (FIGS. **6C****,D**).

A recurrent CPC subset in all three major population is the CD34+CD133+KDR+ or triple-positive subset. These cells have been previously described, after preselection of CD34⁺ cells, and were shown to harbor EPC. In the hands of the present inventors these cells did not contribute to EC formation *in vivo,* possibly due to their low frequency.

Within the KDR⁺ population three more EPC subsets were identified: CD34⁺CD133⁻KDR⁺ cells, CD34⁻CD133⁻KDR⁺ cells and CD34⁻CD133⁺KDR⁺ cells. CD34⁺KDR⁺ cells have been described as potential hematopoietic stem cells or adult hemangioblasts. CD133 is a marker of primitive progenitors but not of mature endothelial cells. Therefore, the CD34⁺CD133⁻KDR⁺ cells detected may be more matured cells. The significance of CD34⁻CD133⁻KDR⁺ cells is as yet difficult to determine, since KDR is expressed on a variety of progenitor cells. The potential identity of CD34⁻CD133⁺KDR⁺ cells can be inferred from the observation that CD133⁺KDR⁺ cells are EPC recruited to the circulation upon vascular trauma. Moreover, this subset resembles - as far as expression of these three markers is concerned - a mesenchymal stem cell population from the bone marrow described by Reyes *et al.* (Reyes M., et al. Origin of endothelial progenitors in human postnatal bone marrow. J. Clin. Invest. 109:337-46, 2002), who have also demonstrated the endothelial cell forming capacity of these cells. Irrespective of their phenotype, however, the combination of these four subsets (i.e., CD34⁺/CD133⁺/KDR⁺, CD34⁻ /CD133⁺/KDR⁺, CD34⁻/CD133⁻/KDR⁺, and CD34⁺/CD133⁻/KDR⁺) did not result in EC differentiation as demonstrated in Example 3 (FIGS. **2** and **3**).

Similarly to KDR⁺ cells, CD133⁺ cells alone do not form endothelial cells *in vivo.* The CD133⁺ population shares with the KDR⁺ population the CD34⁻CD133⁺KDR⁺ subset, previously discussed. The remaining two subsets within the CD133⁺ population are CD34⁻ CD133⁺KDR⁻ and CD34⁺CD133⁺KDR⁻. CD34⁻CD133⁺ cells may be precursors of CD34⁺CD133+ cells, based on their capacity to give rise to CD34⁺ hematopoietic progenitor cells. For this latter phenotype (CD34⁺CD133⁺), functions of hematopoietic progenitor cells, EPC and vascular lymphatic cell progenitors have been proposed. However, in the *in vivo* system described in Example 3 the combination of the four CD133⁺ PC subsets did not give rise to endothelial cells, suggesting that the constellation of factors may not have been adequate to induce endothelial cell differentiation.

A summary of the phenotype and behavior of the seven CPC subsets is found below in Table 1.

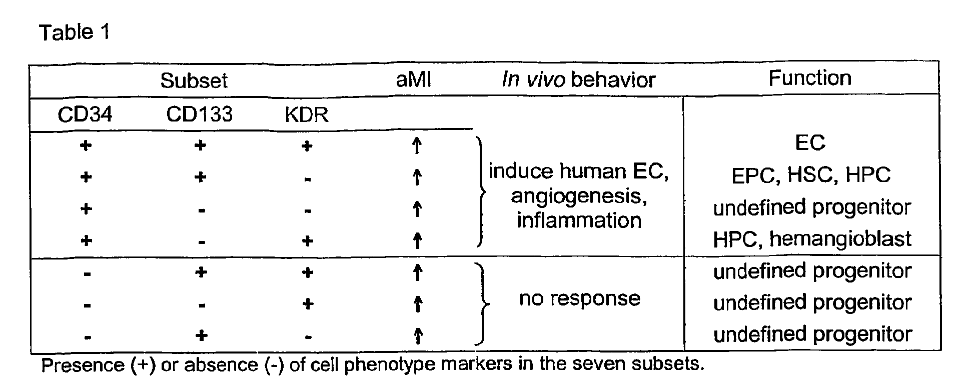

The CD34⁺ population was the only one of the seven identified CPC subpopulations to form mature EC *in vivo,* as demonstrated by binding to the lectin *Ulex europeus-1* agglutinin (UEA-1), expression of CD34⁺ and CD31⁺, spindle shape and organization in networks. The CD34⁺ population shares with the CD133⁺ population the CD34⁺CD133⁺KDR⁻ subset and with the KDR⁺ population the CD34⁺CD133⁻KDR⁺ subset. Whereas these two subsets did not contribute to endothelial cell differentiation in the context of the CD133⁺ and KDR⁺ populations respectively, they did do so in combination with the CD34⁺CD133⁻KDR⁻ subset, which is unique to the CD34⁺ population. Expression of CD34 on peripheral blood mononuclear cells (MNC) was the criterion by which Asahara *et al.* (Asahara T. et al., Isolation of putative progenitor endothelial cells for angiogenesis. Science 275:964-967, 1997) observed that cells with this phenotype, if grown on fibronectin and under angiogenic conditions, could give rise to endothelial cells.

The *in vivo* endothelial cell-forming capacity of CD34⁺ cells is due to the presence of CD34⁺CD133⁻KDR⁻ cells, and optionally, expression of Tie-2 within this subset. The combined presence of CD34⁺CD133⁻KDR⁺ cells and CD34⁺CD133⁺KDR⁻ cells, possibly in combination with the CD34⁺CD133⁻KDR⁻ subset, may be required for endothelial differentiation.

Unexpectedly, CD34⁺ CPC subsets, besides differentiating into EC, also stimulated ingrowth of murine blood vessels into Matrigel^{®}. Although angiogenic by its composition of extracellular matrix components and growth factors, Matrigel^{®} itself (bare Matrigel^{®}) did not induce ingrowth of murine blood vessels during a 14 day observation period (Example 3 and Example 5).

The implantation of CD34⁺ CPC results in neovascularization in two ways. First, human CD34⁺ CPC differentiate into human endothelium. Secondly, the human CD34⁺ CPC induce vascular ingrowth by the host. Not all the CD34+ CPC subsets induced host neovascularization equally. The CD34⁺CD133⁻KDR⁻ subset and the CD34⁺CD133⁺KDR⁻ subset were important for the induction of host neovascularization. Large vessels were primarily seen in the CD34⁺CD133⁻KDR⁻ subset and to a lesser extent in the CD34⁺CD133⁺KDR⁻ subset. Furthermore, a combination of these two subsets was not synergistic and did not lead to higher levels of neovascularization than either subset alone. Additionally, the combination of the CD34⁺CD133⁻KDR⁻ and the CD34⁺CD133⁻KDR⁺ subsets resulted in only the formation of capillaries and not in formation of small and large blood vessels. Induction of primarily capillaries may be useful in treating ischemic heart disease.

Thus far, CPC have been viewed as cells that could directly give rise to new blood vessels and thus to contribute to neovascularization after damage. The unexpected observation by the present inventors demonstrate that CPC can exert a modulatory function on local vasculature, enhancing sprouting angiogenesis. This finding provides new perspectives for improved therapeutic neovascularization.

CD34⁺ CPCs isolated according the teachings of the present invention recruit inflammatory cells of the monocyte/macrophage lineage to the Matrigel^{®} microenvironment. Bare Matrigel^{®} exerted little attraction of murine macrophages, indicating that only a low-grade foreign body reaction against Matrigel^{®} was mounted. Since the inflammatory responses to Matrigel^{®} loaded with CD133⁺ cells or KDR⁺ cells did not exceed those of bare Matrigel^{®}, these subsets did not modulate macrophage infiltration by themselves. In comparison, macrophage infiltration of Matrigel^{®} loaded with CD34⁺ cells was markedly higher, indicating that an additional macrophage-attracting effect of these cells was superimposed on the effect of Matrigel^{®}. Therefore the function of CPC may stretch beyond that of differentiation to endothelial cells and may have additional therapeutic implications. Progenitor cells recruited by damage signals from the ischemic myocardium may not only contribute to neovascularization by directly differentiating to endothelial cells and promoting sprouting of local blood vessels, but may also recruit inflammatory cells to the damaged area.

Pro-inflammatory chemoattractants are produced by the CD34⁺ progenitor cells. While all three CPC subsets, CD34⁺, CD133⁺ and KDR⁺, contained transcripts for the inflammation-associated cytokines/chemokines tumor necrosis factor-α (TNF-α) and macrophage inflammatory protein-1α (MIP-1α), only the CD34⁺ subpopulation responsible for recruiting inflammatory cells expressed high levels (3-fold increased over KDR⁺ cells) of human interleukin-8 (lL-8). Moreover, expression of human lL-8 by single cells persisted for 14 days after Matrigel^{®} implantation.

The surprising observations by the present inventors demonstrates a need for revising the existing definition of CPC, which has previously been based solely on expression of markers such as CD34, CD133 and KDR, because various subsets with varying expression patterns of these molecules exist, which are not equally able to differentiate into endothelial cells. Acute Ml leads to a mobilization of all detected progenitor cell subtypes, demonstrated by similar gene expression patterns in CPC subsets from healthy individuals and aMl patients, indicating that CPC do not respond adaptively to damage signals but rather are passively released from the bone marrow. Finally, CD34⁺ progenitor cells harbor angiogenic as well as immunomodulatory potential, which may be exploited for the generation of new therapeutic strategies using the teachings of the present invention.

Four-parameter fluorescence activated cell sorting is used to identify CPC which yield both blood-vessel forming cells and inflammation mediated cells. The four parameters are lineage, CD34, CD133 and KDR.

A source of cells containing the desired cell population is separated into a desired population and an undesired population by exposing the cells to a cocktail, or mixture, of antibodies, either monoclonal or polyclonal, that define the desired cells. The antibodies are conjugated with fluorescent labels which allow a fluorescence-activated cell sorter to identify cells to which one or more of the antibodies have bound. Individual antibodies can be conjugated with a variety of fluorescent labels (fluorochromes) which are well known to those persons skilled in the art. The antibodies can be linked to one or more than one fluorochrome having the same or unique fluorescence emission wavelengths. Each fluorescence emission wavelength corresponds to a color. Exemplary fluorochromes include, but are not limited to, Texas Red^{®} (Molecular Probes, Eugene, OR), allophycocyanin, phycoerythrin, fluorescein isothiocyanate, rhodamine, SpectralRed^{®} (Southern Biotech, Birmingham, AL), Cy-Chrome, and others.

A source of endothelial progenitor cells or circulating progenitor cells are contacted with a cocktail of antibodies that define lineage-committed (Lin⁺) cells. This cocktail of antibodies are all conjugated to the same fluorochrome. The lineage-committed markers include, but are not limited to, CD3, CD8, CD10, CD14, CD16/56, CD19, CD20, CD31 and CD33. Lin⁺ cells express CD3, CD14, CD16/56, CD19 and CD31. Lineage-uncommitted (progenitor, Lin⁻) cells do not express CD3, CD14, CD16/56, CD19 and CD31.

Lin⁻ cells are isolated by contacting a source of CPC with a cocktail of fluorochrome-labeled antibodies and sorting the cells on a fluorescence activated cell sorter such that a sterile purified population of cells is obtained. Protocols and methods for fluorescence-activated cell sorting are readily available and well known to persons skilled in the art.

Isolated progenitor cells are obtained by contacting Lin⁻ cells with fluorochrome-labeled antibodies to CD34, CD133 and KDR and sorting the labeled cells to identify a population of Lin⁻ cells expressing CD34 but not expressing CD133 or KDR. This sorting step is conducted under sterile conditions.

The isolated progenitor cells are useful for inducing new blood vessel formation in a patient. New blood vessels can be formed by vasculogenesis (formation of blood vessels from embryonic precursors), angiogenesis (in-growth of blood vessels from the surrounding tissue) or the formation of neovascularization (formation of new blood vessels where they had not been previously) including forming blood vessels from endothelial progenitor cells linking to existing blood vessels. There are numerous conditions in which a mammal may be in need of forming new blood vessels such as injury due to trauma, surgery or acute or chronic diseases. In a non-limiting example, the mammal may have a wound that requires healing. In another non-limiting example, the patient may have undergone cardiovascular surgery, cardiovascular angioplasty, carotid angioplasty, or coronary angioplasty, which are all conditions requiring new blood vessel formation. In another non-limiting example, patients who have had a myocardial infarction, such as an aMl, are in need of new blood vessel formation. Other conditions which may require new blood vessel formation include sickle cell anemia and thalassemia.

The isolated progenitor cells can be administered to the mammal in need of forming new blood vessels by any route or method that allows the preferential migration of the cells to the site in need of new blood vessel formation. Exemplary routes of administration include, but are not limited to, systemic administration such as intravenous injection, localized implantation such as localized intramuscular or subcutaneous injection of the progenitor cells in biocompatible solutions or biodegradable biocompatible matrices. Biocompatible solutions are known to those skilled in the art. Examples of biodegradable biocompatible matrices include, but are not limited to, solubilized basement membrane, autologous platelet gel, collagen gels or collagenous substrates based on elastin, fibronectin, laminin, extracellular matrix and fibrillar proteins.

These examples are meant to illustrate one or more embodiments of the present invention and are not meant to limit the invention to that which is described below.

### Example 1

### Identification of seven CPC subsets by four-parameter flow cytometry

The phenotypic heterogeneity of endothelial progenitor cells (EPC) based on patterns of combined expression of three circulating progenitor cells (CPC) markers, CD34, CD133 and KDR was analyzed using four-parameter (three-color) flow cytometric analysis.

Mononuclear cells were isolated from heparinized blood by lymphoprep density gradient centrifugation (Nycomed, Oslo, Norway). Because the number of circulating progenitor cells (PC), irrespective of phenotype, is low, lineage-negative (Lin⁻) cells, i.e. uncommitted, potential PCs, were enriched from total peripheral blood mononuclear cells (MNC) by high-speed flow cytometry sorting, whereas Lin+ cells were discarded (FIG. **6A**). Total MNC were stained with a cocktail of phycoerythrin (PE)-labeled monoclonal antibodies (moAbs) against CD3 (T cells), CD14 (monocytes), CD19 (B cells), CD16/56 (NK cells) and CD31 (mature endothelial cells) (all from IQ Corp., Groningen, The Netherlands). Lin⁻ cells were sorted in basal endothelial medium (Becton Dickinson, Erembodegem-Aalst, Belgium) by high speed flow cytometry using a MoFlo cell sorter (Cytomation, Fort Collins, CO). The obtained Lin⁻ populations were typically 95-98% free of Lin⁺ cells and accounted for an average 11.3 % of all MNC (range 0.6-22.4%), whereas in healthy controls (hc, n=9) an average 3.0% of the MNC were Lin⁻ (range 1.0-5.5%; P=0.0003) (Figure **6A**).

To determine expression patterns of the CPC markers CD34, CD133 and KDR, sorted Lin⁻ cells were subjected to three-color staining using CD34-allophycocyanin (APC) (clone 581, IQ Corp.), CD133-PE (Miltenyi Biotech, Germany) and rabbit polyclonal anti-KDR-fluorescein isothiocyanate (FITC) (Sigma Chemical Co.).

Within the Lin⁻ population, cells expressing one of the three CPC markers CD34, CD133 and KDR were gated, followed by analysis of the expression of the remaining two markers (FIG **6B****:** CD34, FIG **6C**: CD34, FIG **6D****:** KDR). Using this approach, seven CPC subsets were detected based on combined expression of CD34, CD133 and KDR. Triple-negative cells were not considered EPC. All seven subsets were present in aMl patients and healthy controls.

The CD34⁺ population consisted mainly of CD34⁺CD133⁺KDR⁻ cells (aMl, mean 62% of all CD34+ cells, range 33-89%; healthy controls, mean 38%, range 24-50%) and CD34⁺CD133⁻KDR⁻ cells (aMl, mean 37% of all CD34⁺ cells, range 10-61%; healthy controls, mean 60%, range 0.1-0.8%), whereas the triple-positive subset and CD34⁺CD133⁻KDR⁺ subset accounted for less than 1% of this subpopulation (FIG. **6C**).

In the CD133⁺ population in aMI patients, CD34⁺CD133⁺KDR⁻ cells (mean 52% of all CD133⁺ cells, range 33-89%) and CD34⁻CD133⁺KDR⁻ cells (mean 28% of all CD133⁺ cells, range 0.1-85%) dominated, whereas in healthy controls CD34⁺CD133⁺KDR⁻ cells (mean 38%, range 13-76%, one outlier 1%) and CD34⁻CD133⁺KDR⁺ cells (mean 58%, range 12-73%) were the dominating subsets (FIG. **6B**).

Differences between aMI patients and healthy controls were also present in the KDR⁺ population, which in aMl patients consisted mainly of CD34⁻CD133⁻KDR⁺ cells (mean 65%, range 1-95%) and CD34⁻CD133⁺KDR⁺ cells (mean 33%, range 3-71%) (FIG. **6D**). In healthy controls CD34⁻CD133⁺KDR⁺ cells dominated (mean 93%, range 52-99%), whereas the CD34⁻CD133⁻KDR⁺ subset encompassed only about 5% of all KDR⁺ cells (FIG. **6D**).

### Example 2

### CPC numbers in aMI patients and healthy controls

Ten aMl patients and nine healthy control volunteers were compared with regard to EPC numbers to determine whether the number of cells in the seven CPC subsets correlated with the event of aMl. A possible correlation between CPC numbers and aMl is reflected in the number of Lin⁻ cells (within which CPC were detected). Numbers of Lin⁻ cells were compared between the two subject groups. In aMl patients the number of Lin⁻ cells averaged 2.6x10⁵ cells/mL blood (range 0.2-4.7x10⁵ cells/mL blood), which was significantly higher (P=0.001) than in healthy controls (mean 0.5x10⁵ cells/mL blood, range 0.04-1.4x10⁵ cells/mL blood) (FIG. **7**), equivalent with a 5.2-fold higher number of Lin⁻ cells in aMl patients as compared to controls.

The numbers of CPC expressing CD34, CD133 or KDR were compared in aMI patients and healthy controls. CPC numbers in all three subsets were significantly higher in aMl patients than in healthy controls. In the CD34 subset an 8.6-fold higher cell number was found in aMI patients (mean 2.6x10⁴ cells/mL blood, range 2.1-11.0x10⁴ cells/mL blood, P=0.005) relative to healthy controls (mean 0.3x10⁴ cells/mL blood, range 0.06-1.2x10⁴ cells/mL blood). In the CD133 subset an 11.6-fold higher cell number was found in aMl patients (mean 3.5x10⁴ cells/mL blood, range 0.5-13.5x10⁴ cells/mL blood, P<0.0001) relative to healthy controls (mean 0.3x10⁴ cells/mL blood, range 0.07-0.7x10⁴ cells/mL blood). Finally, in the KDR subset a 6.2-fold higher cell number was found in aMl patients (mean 1.3x10⁴ cells/mL blood, range 0.03-5.8x10⁴ cells/mL blood, P=0.005) relative to healthy controls (mean 0.2x10⁴ cells/mL blood, range 0.009-0.8x10⁴ cells/mL blood).

To establish whether aMl triggered the mobilization of a specific CPC subset, possibly for repair of damaged myocardial blood vessels, the number of CPC within the seven subsets was determined. In all seven subsets, irrespective of their phenotype, significantly higher CPC numbers were present in aMl patients than in healthy controls (FIG. **8**). At the patient level, outliers in CPC numbers in specific subsets were readily apparent, although in these patients CPC numbers were not consistently higher in all seven CPC subsets.

Because numbers of CPC, irrespective of their phenotype, were increased in aMl patients, suggesting a causal link between cardiovascular damage and CPC recruitment, correlations between CPC numbers and disease parameters were sought (Table 2). Fifteen aMl patients and 10 patients with stable angina pectoris were included in this analysis. Cardiovascular disease (CVD) history indicates previous episodes of CVD in patients. Number of aMl indicates the number of the current aMI episode. In addition to the risk factors for aMl listed in Table 2, age (>60 years) and male gender were considered risk factors for aMl, resulting in a total of six possible risk factors. The cumulative risk factors indicate the number of risk factors out of these six possible risk factors, present in a given patient.

There was no correlation between CPC numbers in various subsets and age, cumulative number of risk factors or ischemic time. Moreover, there was also no correlation between EPC numbers and serum lactate dehydrogenase (LDH), creatinine phsophokinase myocard band (CKMB) or troponin.

**Table 2. Demographic and post aMI clinical characteristics of aMI patients**

| | | | | | Ml risk factors | | | | | | | | | |
|---|---|---|---|---|---|---|---|---|---|---|---|---|---|---|
| Indic | age | gender | CVD | aMl | | CVD | | | cum. | ischemic | LDH | CKMB | troponin | post aMI |
| | | | history | number | smoking | family | hyper- | hyper- | risk | time (h) | (U/I) | U/I) | Ug/l) | medication |
| | | | | | | history | tens. | cholest. | factors | | | | | |
| aMI | 48 | m | 1 | 2 | yes | no | no | yes | 3 | 3.2 | 1600 | 165 | 2392 | B,AS,S,O |
| aMI | 45 | m | 0 | 1 | yes | yes | no | yes | 4 | 2.67 | 473 | 63 | 40 | B,AS,S |
| aMI | 41 | m | 1 | 1 | yes | yes | no | no | 3 | 3 | 991 | 168 | 733.7 | B,AS,S |
| aMI | 55 | m | 0 | 1 | yes | no | no | no | 2 | 5 | 573 | 54 | 346 | B,N,AS,S |
| aMI | 43 | m | 1 | 1 | yes | yes | yes | yes | 5 | 7 | 940 | 80 | 216.4 | B,AS,S,O |
| aMI | 69 | f | 0 | 1 | yes | yes | no | yes | 4 | 4 | 592 | 88 | 0.2 | B,AS,S,O |
| aMI | 63 | f | 1 | 1 | yes | yes | no | yes | 4 | 8.5 | 825 | 97 | 503.6 | B,AS,S |
| aMI | 52 | m | 0 | 1 | yes | no | no | no | 2 | 6.5 | 829 | 86 | 27.1 | B,AS |
| aMI | 59 | m | 1 | 1 | no | no | yes | no | 2 | 6.5 | 1975 | 264 | 1392 | B,AS |
| aMI | 56 | f | 1 | 1 | yes | no | yes | n | 2 | 6.5 | 716 | 46 | 83.6 | B,AS,S,O |
| aMI | 58 | m | 1 | 1 | no | yes | no | no | 2 | 2 | 924 | 125 | 1085.6 | B,AS,AI,S |
| aMI | 58 | f | 0 | 1 | yes | yes | no | yes | 3 | 2 | 427 | 48 | 176.4 | B,AS,S |
| aMI | 40 | m | 0 | 1 | yes | no | no | yes | 3 | 2 | 907 | 97 | 352.3 | B,AS,AI,S |
| aMI | 57 | m | 0 | 1 | no | yes | no | yes | 3 | 2 | 219 | 3 | 0 | AS,S |
| aMI | 44 | m | 0 | 1 | yes | yes | no | yes | 4 | 3 | 1144 | 114 | 1255.2 | B,AS,S |
| sAP | 51 | m | 1 | 1 | no | yes | no | yes | 2 | - | - | - | - | B,AS,S,C,AI,TI |
| sAP | 58 | m | 1 | 0 | no | yes | yes | yes | 4 | - | - | - | - | B,AS,S,C,AI |
| sAP | 55 | m | 1 | 1 | ? | ? | no | no | ? | - | - | - | - | AS,S |
| sAP | 64 | m | 1 | 1 | yes | yes | no | yes | 3 | - | - | - | - | B,AS,S,AI,O |
| sAP | 67 | m | 1 | 0 | no | no | no | no | 0 | - | - | - | - | B,S |
| sAP | 71 | f | 1 | 1 | no | yes | no | yes | 2 | - | - | - | - | B,AS,S,C,ARB,O |
| sAP | 62 | m | 1 | 0 | no | yes | no | no | 1 | - | - | - | - | AS,Tl,S,C,AI |
| sAP | 53 | m | 1 | 1 | no | no | yes | yes | 2 | - | - | - | - | B,AS,S,AI |
| sAP | 63 | m | 1 | 0 | no | yes | yes | yes | 4 | - | - | - | - | B,S,C,O |
| sAP | 72 | m | 1 | 0 | no | yes | no | yes | 3 | - | - | - | - | B,S,O |

| | | | | | | | | | | | | | | |
|---|---|---|---|---|---|---|---|---|---|---|---|---|---|---|
| Post aMI medication: beta blockers (B), acetyl salicylate (AS), nitroglycerine (N), ace inhibitor (AI), statins (S), ticlopidin (TI), Ca antagonist (C), angiotensin receptor blocker (ARB) or others (O). Ischemic time = period between onset of chest pain and intervention. | | | | | | | | | | | | | | |

### Example 3

### Blood vessel-forming activity of CPC subsets

This experiment investigated the behavior of CPC subsets *in vivo,* primarily with respect to differentiation into mature endothelial cells (EC). CPC expressing either CD34, CD133 or KDR were sorted in 200 µL Matrigel^{®} and supplemented with 10 ng basic fibroblast growth factor (b-FGF, Chemicon, Temecula, CA) and 12 U heparin (Leo Pharma, Ballerup, Denmark) at 5,000 to 15,000 cells per implant and implanted subcutaneously in nude mice. Bare Matrigel^{®} contains the b-FGF and heparin supplement. After 14 days, the Matrigel^{®} pellets were explanted, partly snap-frozen in liquid nitrogen for immunohistochemistry, or fixed in 2% paraformaldehyde in 0.1 M sodium phosphate buffer, dehydrated and embedded in resin (Technovit 8100, Heraeus Kulzer, Wehrheim, Germany). For overall morphologic evaluation, 2 µm sections of resin-embedded Matrigel^{®} pellets were stained with toluidin blue. Morphologic analysis of the implants showed that by 14 days after implantation, high cellularity was present in Matrigel^{®} seeded with CD34⁺ cells (FIG. **2B**). Cellularity was markedly lower in CD133⁺-loaded Matrigel^{®} (FIG. **2C**) and minimal in Matrigel^{®} seeded with KDR⁺ cells or bare Matrigel^{®} (FIG. 2A). Network structures composed of spindle shaped cells, strongly resembling capillary networks, were abundant in Matrigel^{®} seeded with CD34⁺ cells but scarce or virtually absent in Matrigel^{®} seeded with CD133⁺ cells, KDR⁺ cells, or bare Matrigel^{®}.

To determine whether these network structures were formed by human CPC, Matrigel^{®} sections were stained with UEA-1, which binds specifically to human, but not to murine, EC (FIGS. **3A,B**). Human umbilical vein endothelial cells (HUVEC) were used as a positive control for UEA-1 staining (FIG. **3A**) and H5V cells (murine EC line) as a negative control (FIG. **3B**). Network structures in CD34⁺ implants were positive for UEA-1 (FIG. **3C**); however, UEA-1-positive cells were not present in Matrigel^{®} seeded with the other subsets or in bare Matrigel^{®}.

Because the UEA-1 staining demonstrated the human origin of network structures in Matrigel^{®} *in vivo,* but cannot differentiate between progenitor and mature EC, the explants were stained with the EPC/EC marker CD34 and the EC maturation marker CD31. In Matrigel^{®} seeded with CD34+ EPC, spindle-shaped cells, occasionally arranged in network structures and similar to the cells observed after staining with UEA-1, were positive for human CD34 (FIG. **3D**). CD31 was present on cells with similar morphology in Matrigel^{®} seeded with CD34⁺ cells (FIG. **3E**). Neither CD34, nor CD31 was detectable in Matrigel^{®} seeded with CD133⁺ CPC, KDR⁺ CPC or bare Matrigel^{®}.

### Example 4

### Induction of angiogenic and inflammatory responses to CPC subsets in vivo

Because not all cells detected in Matrigel^{®} seeded with CD34⁺ cells stained for human EC markers, it was investigated whether they were murine EC. Using immunohistochemistry, murine blood vessels and inflammatory cells were detected using rat monoclonal antibodies directed against murine CD31 (Southern Biotech, Birmingham, AL) and monocytes/macrophages (MoMa, Serotech, Oxford, UK). Strong host-derived angiogenic responses were detected towards Matrigel^{®} seeded with human CD34⁺ cells (FIGS. **4B,E**). However, Matrigel^{®} containing human CD133⁺ cells triggered weaker murine angiogenic responses (FIGS. **4C,F**) whereas the angiogenic reaction upon implantation of Matrigel^{®} seeded with KDR⁺ cells or bare Matrigel^{®} was marginal (FIGS. **4A,D**). A strong influx of murine MoMa⁺ inflammatory cells towards human CD34⁺ cells and weak responses against the other 2 subsets (FIGS. **4D-F**) were observed in all 5 tested subjects (2 aMl patients, 3 healthy controls) and did not differ between CPC from aMl patients and healthy controls. No differentiation towards human monocytes (CD14⁺ cells) or macrophages (CD68⁺, FIG. **4E** inset) was detected.

### Example 5

### Effects of CD34⁺ subsets on Angiogenesis

CD34⁺ CPC were isolated as described in Example 1 and propidium iodine staining was included to ensure that only living cells were isolated (FIG. **9**). Four CD34⁺ subsets were further isolated from the CD34⁺ population: CD34⁺CD133⁻KDR⁻ (+--); CD34⁺CD133⁺KDR⁻ (++-); CD34⁺CD133⁻KDR⁺ (+-+); and CD34⁺CD133⁺KDR⁺ (+++), The four CD34+ CPC subsets were resuspended in supplemented Matrigel^{®} and implanted in nude mice as described in Example 3. Single subsets or mixed populations were implanted in mice according to the Experimental Setup in Table 3.

**Table 3**

| **Experimental Group** | **Injected Subset(s) CD34/CD133/KDR** | **N** |
|---|---|---|
| 1 | ++- | 3 |
| 2 | +-- | 3 |
| 3 | ++-/+-- | 3 |
| 4 | +++/+-- | 3 |
| 5 | +--/+-+ | 3 |

For overall histologic evaluation, 2 µm sections were prepared from the resin-embedded Matrigel^{®} pellets and stained with toluidin blue. The number of blood vessels were counted and corrected for the area of investigated tissue, resulting in the number of vessels per square micrometer. Blood vessels are defined as follows: large vessels containing erythrocytes, surrounded by smooth muscle; medium vessels consisting of erythrocytes and smooth muscle; and capillaries.

The specific binding of lectins to endothelium was used to detect the presence of both human and murine endothelial cells. The lectin UEA-1 binds specifically to human endothelium and BS-1 lectin (*Bandeiraea simplicifolia-1,* Sigma) selectively binds to murine endothelium. Additionally, antibodies directed specifically against human and murine CD31, a marker for endothelial cells, were used. Detection was achieved using the ABC kit (Vector labs) and amino-ethyl carbazole (AEC). The number of CD31-positive cells was counted and corrected for the area of tissue.

CD34⁺ CPC-containing Matrigel^{®} had increased vascularization when compared to bare Matrigel^{®}. Staining these Matrigel^{®} pellets for human CD31 demonstrated the presence of large CD31-positive cell clusters representing immature endothelial cells (FIG. 10A). These clusters were present primarily in the CD34⁺CD133⁻KDR⁻ and CD34⁺CD133⁺KDR⁻ subsets. A small number of human CD31-positive cells associated with vessel-like structures were seen in the CD34⁺CD133⁻KDR⁻ subset (FIG. **10B**).

While the human CPCs had not differentiated into human blood vessels, the CD34⁺ human CPC subsets did provide and inductive effect of host murine neovascularization. There was an increased incidence of murine CD31-positive vasculature in the CD34+ CPC-loaded Matrigel^{®} with vessels ranging in size from capillaries to large vessels (FIG. **11**).

The number of murine CD31-positive vessels/mm² (including capillaries, small and large vessels was determined for five experimental groups listed in Table 3 (FIG. 12). The criteria for identification of blood vessels are: capillaries have 1-2 endothelial cells; small vessels have 3-5 endothelial cells; and large vessels have more than 5 endothelial cells and may also have vascular smooth muscle surrounding the vessel. The CD34+ CPC subsets induced higher levels of host murine vascularization over bare Matrigel^{®} controls. Highest levels of vascularization were seen in the CD34⁺CD133⁻KDR⁻ and CD34⁺CD133⁺KDR⁻ subsets. Large vessels were primarily seen in the CD34⁺CD133⁻KDR⁻ subset and to a lesser extent in the CD34⁺CD133⁺KDR⁻ subset. Furthermore, a combination of these two subsets was not synergistic and did not lead to higher levels of neovascularization than either subset alone. Additionally, the combination of the CD34⁺CD133⁻KDR⁻ and the CD34⁺CD133⁻KDR⁺ subsets resulted in only the formation of capillaries and not in formation of small and large blood vessels.

### Example 6

### CPC transcription profiles of aMl patients and healthy controls

Previous studies indicate that CPC can express a panel of markers related to their development and function. Although the above described four-parameter flow cytometric analysis allows simultaneous assessment of three CPC markers on single CPC, this approach does not cover all CPC markers known so far. Therefore quantitative RT-PCR was used to investigate the presence and expression level of a number of transcripts related to CPC development, maturation and function and to determine which factors mediated the observed pro-angiogenic and pro-inflammatory effects seen in CD34⁺ CPC.

Total RNA was isolated from 10³-10⁴ CPC from the desired phenotype (CD34⁺, CD133⁺ and KDR⁺) and random hexamers and copy DNA was synthesized. Primer/probe sets (TaqMan, Applied Biosystems, Foster City, CA) for human GAPDK, beta-2-microglobulin (B2M), beta-actin, c-abl, CD34, CD133, Tie-1, Tie-2, flt-1, KDR, VEGF, CD31, VE-cadherin, von Willebrand factor (vWF), interleukin-8 (lL-8), tumor necrosis factor-α (TNF-α), granulocyte macrophage colony stimulating factor (GM-CSF), macrophage inflammatory protein-1α (MIP-1α), macrophage chemoattractant protein-1 (MCP-1), MCP-2 and MCP-3 were used for CPC transcript analysis. Triplicate RT-PCR reactions were performed on equal amounts of cDNA using the following parameters: 2 min 50°C, 10 min 95°C, and 45 cycles consisting of 15 sec denaturation (95°C) and 1 min annealing/extension (60°C). The variation (SD) of combined cDNA synthesis and PCR was less than 0.5 C_{T} (cycle threshold) for the GAPDH housekeeping mRNA. Cycle threshold values were normalized to beta-2-microglobulin using the ΔC_{T} method and differences in expression levels between patients and controls or between subsets are expressed as fold variance of expression, calculated as 2^{-ΔΔCT} (Livak KJ et al. Analysis of relative gene expression data using real-time quantitative PCR and the 2-ΔΔC Method. Methods. 25:402-8, 2001).

The results of RT-PCT to determine the presence of mRNA transcripts of 14 markers potentially involved in CPC maturation and function are presented in Table 4. Because there was inter-individual variance with respect to gene expression, the expression of a gene in a given CPC subset was defined as the presence of a PCR product (i.e. a C_{T} value < 45) in at least 3/5 subjects. Based on this definition, gene expression profiles in aMI patients and healthy controls were similar.

**Table 4. Gene expression profiling in CPC subsets**

| | **Healthy control** | | | | **aMI** | | **HUVEC** |
|---|---|---|---|---|---|---|---|
| **Marker** | **KDR** | **CD133** | **CD34** | **KDR** | **CD133** | **CD34** | |
| GAPDH | + | + | + | + | + | + | + |
| B2M | + | + | + | + | + | + | + |
| B-Act | + | + | + | + | + | + | + |
| c-abl | n.d. | + | + | + | + | + | + |
| Tie-1 | n.d. | + | + | n.d. | + | + | + |
| Tie-2 | n.d. | n.d. | + | n.d. | n.d. | + | + |
| CD34 | n.d. | + | + | n.d. | + | + | + |
| CD133 | n.d. | + | + | n.d. | n.d. | + | n.d. |
| flt-1 | + | n.d. | + | + | n.d. | + | + |
| KDR | n.d. | n.d. | n.d. | n.d. | n.d. | n.d. | + |
| VEGF | + | + | + | + | + | + | + |
| CD31 | + | + | + | + | + | + | + |
| VE-cadh | n.d. | n.d. | n.d. | n.d. | n.d. | n.d. | + |
| vW F | n.d. | + | + | + | + | + | + |

| | | | | | | | |
|---|---|---|---|---|---|---|---|
| 10³-10⁴ CPC were sorted from 5 aMl patients and 5 healthy controls. When a PCR product was detected in at least 3/5 individuals, gene expression was considered to be present in the respective subset (+). n.d. = not detected. RNA isolated from 1000 HUVEC (human umbilical vein endothelial cells) was used as a positive control. | | | | | | | |

When comparing the three CPC subsets, gain of gene expression was apparent in the order KDR → CD133 → CD34. Tie-1, CD34 and, in healthy controls, CD133 and vWF transcripts were present in the CD133 subset, but not in the KDR subset. Fit-1 transcripts, which were detectable in the KDR⁺ subset, were absent in CD133⁺ cells. In the CD34⁺ subset, transcripts of Tie-2, flt-1, CD133 and, in aMl patients, CD34 were gained in comparison to KDR⁺ and CD133⁺ cells.

Transcripts of the inflammation-associated molecules GM-CSF, MCP-1, CMP-2 and MCP-3 were below PCT detection limits in all subsets. TNF-*α* and MIP-1*α* transcripts were present in similar amounts in CD34⁺ CPC and KDR⁺ CPC. Interleukin-8 (lL-8) transcripts were present in CD34⁺ transcripts from all included individuals. In KDR⁺ CPC, however, lL-8 transcripts were found in only 2 of 5 individuals. Moreover, lL-8 transcript levels were 3-fold higher in the CD34⁺ subsets than in the KDR⁺ subset. Interleukin-8 transcripts were found in CD34⁺ CPC both directly after sorting and after 14 day implantation in Matrigel^{®} in nude mice (FIG. **5**).

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Of course, variations on those preferred embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law.

Furthermore, numerous references have been made to patents and printed publications throughout this specification.

In closing, it is to be understood that the embodiments of the invention disclosed herein are illustrative of the principles of the present invention. Thus, by way of example, but not of limitation, alternative configurations of the present invention may be utilized in accordance with the teachings herein.

## Claims

1. A method for isolation of endothelial progenitor cells comprising:
identifying lineage-committed cells from a source of progenitor cells by contacting said progenitor cells with a plurality of fluorochrome-labeled antibodies specific for the cell markers CD3, CD14, CD16/56, CD19 and CD31;
depleting said lineage-committed cells by fluorescence activated cell sorting to form a population of lineage-negative cells, wherein said lineage-negative cells do not express the cell surface markers CD3, CD14, CD16/56, CD19 and CD31;
reacting said lineage-negative cells with a plurality of fluorochrome-labeled antibodies specific for the cell markers selected from the group consisting of CD34, CD133 and KDR wherein each antibody is labeled with a fluorochrome with a unique emission wavelength; and
sorting said labeled lineage-negative cells by three-color fluorescence activated cell sorting to form a population of endothelial progenitor cells, wherein said endothelial progenitor cells are selected from the group consisting of CD34⁺CD133⁻KDR⁻, CD34⁺CD133⁺KDR⁺, CD34⁺CD133⁺KDR⁻ and CD34⁺CD133⁻KDR⁺ endothelial progenitor cells, excluding the use of human embryos for commercial or industrial processes.

2. The method of claim 1 wherein said source of progenitor cells is a mammalian source.

3. The method of claim 2 wherein said mammalian source is a human source.

4. The method of claim 1 wherein said source of progenitor cells is peripheral blood.

5. The method of claim 1 wherein said endothelial progenitor cells are blood vessel generating cells, inflammation-mediating cells or both.

6. The method of claim 5 wherein said blood vessel-generating cells are CD34⁺CD133⁻KDR⁻ endothelial progenitor cells.

7. The method of claim 5 wherein said inflammation-mediating cells are CD34⁺CD133⁻KDR⁻ endothelial progenitor cells.

8. The method of claim 5 wherein said inflammation-mediating cells express interleukin-8.

9. The method of claim 1 wherein said endothelial progenitor cells induce angiogenic responses in surrounding blood vessels.

10. A therapeutic composition for inducing angiogenesis at a treatment site comprising:
a biodegradable biocompatible matrix having lineage-negative CD34⁺ endothelial progenitor cells disposed therein, wherein said lineage-negative cells do not express the cell surface markers CD3, CD14, CD16/56, CD19 and CD31 and wherein said CD34⁺ endothelial progenitor cells are selected from the group consisting of CD34⁺CD133-KDR⁻, CD34⁺CD133⁺KDR⁺, CD34⁺CD133⁺KDR⁻ and CD34⁺CD133⁻KDR⁺ endothelial progenitor cells, providing the progenitor cells are not of human embryonic origin.

11. The therapeutic composition of claim 10 wherein said CD34⁺ endothelial progenitor cells are CD34⁺CD133⁻KDR⁻ endothelial progenitor cells.

12. The therapeutic composition of 10 wherein said biodegradable biocompatible matrix is selected from the group consisting of solubilized basement membrane, autologous platelet gel, collagen gels or collagenous substrates based on elastin, fibronectin, laminin, extracellular matrix and fibrillar proteins.

13. A therapeutic composition having a chemotactic effect on inflammation mediating cells at a treatment site comprising:
a biodegradable biocompatible matrix having lineage-negative CD34⁺ endothelial progenitor cells disposed therein, wherein said lineage-negative cells do not express the cell surface markers CD3, CD14, CD16/56, CD19 and CD31 and wherein said CD34⁺ endothelial progenitor cells are selected from the group consisting of CD34⁺CD133' KDR⁻, CD34⁺CD133⁺KDR⁺, CD34⁺CD133⁺KDR⁻ and CD34⁺CD133⁻KDR⁺ endothelial progenitor cells, providing the progenitor cells are not of human embryonic origin.

14. The therapeutic composition of claim 13 wherein said CD34⁺ endothelial progenitor cells are CD34⁺CD133⁻KDR⁻ endothelial progenitor cells.

15. The therapeutic composition of 13 wherein said biodegradable biocompatible matrix is selected from the group consisting of solubilized basement membrane, autologous platelet gel, collagen gels or collagenous substrates based on elastin, fibronectin, laminin, extracellular matrix and fibrillar proteins.

16. An isolated population of endothelial progenitor cells wherein said isolated population is lineage-negative and CD34⁺, wherein said lineage-negative cells do not express the cell surface markers CD3, CD14, CD16/56, CD19 and CD31 and wherein said CD34⁺ endothelial progenitor cells are selected from the group consisting of CD34⁺CD133-KDR⁻, CD34⁺CD133⁺KDR⁺, CD34⁺CD133⁺KDR⁻ and CD34⁺CD133⁻KDR⁺ endothelial progenitor cells, providing the progenitor cells are not of human embryonic origin.

17. The isolated population of endothelial progenitor cells of claim 16 wherein said isolated population is lineage-negative and CD34⁺CD133⁻KDR⁻.

18. The isolated population of endothelial progenitor cells of claim 16 wherein said isolated population comprises blood vessel-generating cells.

19. The isolated population of endothelial progenitor cells of claim 16 wherein said isolated population comprises inflammation-mediating cells.

20. The isolated population of endothelial progenitor cells of claim 16 wherein said isolated population induces angiogenic responses in surrounding blood vessels.

## Patentansprüche

1. Verfahren zur Isolierung von Endothelprogenitorzellen, umfassend:
Identifizieren liniendeterminierter Zellen aus einer Quelle von Progenitorzellen durch Kontaktieren der Progenitorzellen mit einer Vielzahl von mit Fluorchrom markierten Antikörpern, die für die Zellmarker CD3, CD14, CD16/56, CD19 und CD31 spezifisch sind;
Depletieren der liniendeterminierten Zellen durch fluoreszenzaktiviertes Zellsortieren zur Bildung einer Population von liniennegativen Zellen, worin die liniennegativen Zellen nicht die Zelloberflächenmarker CD3, CD14, CD16/56, CD19 und CD31 exprimieren;
zur Reaktion bringen der liniennegativen Zellen mit einer Vielzahl von mit Fluorchrom markierten Antikörpern, die für die Zellmarker spezifisch sind, die aus der Gruppe ausgewählt sind, bestehend aus CD34, CD133 und KDR, worin jeder Antikörper mit einem Fluorchrom mit einer einzigen Emissionswellenlänge markiert ist; und
Sortieren der markierten liniennegativen Zellen mithilfe von fluoreszenzaktiviertem Zellsortieren (3 Farben) zur Bildung einer Population von Endothelprogenitorzellen, worin die Endothelprogenitorzellen aus der Gruppe ausgewählt sind, bestehend aus CD34⁺CD133⁻KDR⁻-, CD34⁺CD133⁺KDR⁺-, CD34⁺CD133⁺KDR⁻- und CD34⁺CD133⁻ KDR⁺-Endothelprogenitorzellen, ausschließlich der Verwendung von humanen Embryos für gewerbliche oder industrielle Verfahren.

2. Verfahren nach Anspruch 1, worin die Quelle von Progenitorzellen eine Säugerquelle darstellt.

3. Verfahren nach Anspruch 2, worin die Säugerquelle eine humane Quelle darstellt.

4. Verfahren nach Anspruch 1, worin die Quelle von Progenitorzellen peripheres Blut darstellt.

5. Verfahren nach Anspruch 1, worin die Endothelprogenitorzellen blutgefäßbildende Zellen, entzündungsvermittelnde Zellen oder beides darstellen.

6. Verfahren nach Anspruch 5, worin die blutgefäßbildenden Zellen CD34⁺CD133⁻KDR⁻ -Endothelprogenitorzellen darstellen.

7. Verfahren nach Anspruch 5, worin die entzündungsvermittelnden Zellen CD34⁺CD133⁻KDR⁻ -Endothelprogenitorzellen darstellen.

8. Verfahren nach Anspruch 5, worin die entzündungsvermittelnden Zellen Interleukin-8 exprimieren.

9. Verfahren nach Anspruch 1, worin die Endothelprogenitorzellen angiogene Reaktionen in umgebenden Blutgefäßen induzieren.

10. Therapeutische Zusammensetzung zum Induzieren der Angiogenese an einem Behandlungsort, umfassend:
eine biologisch abbaubare biokompatible Matrix mit liniennegativen CD34⁺-Endothelprogenitorzellen darinnen angeordnet, worin die liniennegativen Zellen nicht die Zelloberflächenmarker CD3, CD14, CD16/56, CD19 und CD31 exprimieren und worin die CD34⁺-Endothelprogenitorzellen aus der Gruppe ausgewählt sind, bestehend aus CD34⁺CD133⁻KDR⁻-, CD34⁺CD133⁺KDR⁺-, CD34⁺CD133⁺KDR - und CD34⁺CD133⁻ KDR -Endothelprogenitorzellen, vorausgesetzt, dass die Progenitorzellen von nicht humaner embryonaler Herkunft sind.

11. Therapeutische Zusammensetzung nach Anspruch 10, worin die CD34⁺-Endothelprogenitorzellen CD34⁺CD133⁻KDR⁻ -Endothelprogenitorzellen darstellen.

12. Therapeutische Zusammensetzung nach Anspruch 10, worin die biologisch abbaubare biokompatible Matrix aus der Gruppe ausgewählt ist, bestehend aus solubilisierter Basalmembran, autologem Thrombozytengel, Kollagengelen oder Kollagensubstraten, die auf Elastin, Fibronektin, Laminin, extrazellulärer Matrix und Faserproteinen basieren.

13. Therapeutische Zusammensetzung mit einer chemotaktischen Wirkung auf entzündungsvermittelnde Zellen an einem Behandlungsort, umfassend:
eine biologisch abbaubare biokompatible Matrix mit liniennegativen CD34⁺-Endothelprogenitorzellen darin angeordnet, worin die liniennegativen Zellen nicht die Zelloberflächenmarker CD3, CD14, CD16/56, CD19 und CD31 exprimieren und worin die CD34⁺-Endothelprogenitorzellen aus der Gruppe ausgewählt sind, bestehend aus CD34⁺CD133⁻KDR⁻-, CD34⁺CD133⁺KDR⁺-, CD34⁺CD133⁺KDR⁻- und CD34⁺CD133 KDR⁺-Endothelprogenitorzellen, vorausgesetzt, dass die Progenitorzellen nicht von humaner embryonaler Herkunft sind.

14. Therapeutische Zusammensetzung nach Anspruch 13, worin die CD34⁺-Endothelprogenitorzellen für CD34⁺CD133⁻ KDR⁻-Endothelprogenitorzellen stehen.

15. Therapeutische Zusammensetzung nach Anspruch 13, worin die biologisch abbaubare biokompatible Matrix aus der Gruppe ausgewählt ist, bestehend aus solubilisierter Basalmembran, autologem Thrombozytengel, Kollagengelen oder Kollagensubstraten, die auf Elastin, Fibronektin, Laminin, extrazellulärer Matrix und Faserproteinen basieren.

16. Isolierte Population von Endothelprogenitorzellen, worin die isolierte Population liniennegativ und CD34⁺ ist, worin die liniennegativen Zellen nicht die Zelloberflächenmarker CD3, CD14, CD16/56, CD19 und CD31 exprimieren und worin die CD34⁺-Endothelprogenitorzellen aus der Gruppe ausgewählt sind, bestehend aus CD34⁺CD133⁻KDR⁻-, CD34⁺CD133⁺KDR⁺-, CD34⁺CD133⁺KDR⁻- und CD34⁺CD133⁻ KDR⁺-Endothelprogenitorzellen, vorausgesetzt, dass die Progenitorzellen nicht von humaner embryonaler Herkunft sind.

17. Isolierte Population von Endothelprogenitorzellen nach Anspruch 16, worin die isolierte Population liniennegativ und CD34⁺CD133⁻KDR⁻ ist.

18. Isolierte Population von Endothelprogenitorzellen nach Anspruch 16, worin die isolierte Population blutgefäßbildende Zellen umfasst.

19. Isolierte Population von Endothelprogenitorzellen nach Anspruch 16, worin die isolierte Population entzündungsvermittelnde Zellen umfasst.

20. Isolierte Population von Endothelprogenitorzellen nach Anspruch 16, worin die isolierte Population angiogene Reaktionen in umgebenden Blutgefäßen induziert.

## Revendications

1. Procédé d'isolation de cellules progénitrices endothéliales, comprenant les étapes consistant à :
identifier des cellules commises vers une lignée à partir d'une source de cellules progénitrices en mettant lesdites cellules progénitrices en contact avec une pluralité d'anticorps marqués par un fluorochrome et spécifiques aux marqueurs cellulaires CD3, CD14, CD 16/56, CD19 et CD31 ;
dépléter lesdites cellules commises vers une lignée par un tri cellulaire activé par fluorescence afin de former une population de cellules négatives pour la lignée, lesdites cellules négatives pour la lignée n'exprimant pas les marqueurs de surface cellulaire CD3, CD 14, CD16/56, CD 19 et CD31 ;
faire réagir lesdites cellules négatives pour la lignée avec une pluralité d'anticorps marqués par un fluorochrome et spécifiques aux marqueurs cellulaires choisis dans le groupe constitué par CD34, CD133 et KDR, chaque anticorps étant marqué par un fluorochrome ayant une longueur d'onde d'émission unique ; et
trier lesdites marquées cellules négatives pour la lignée par un tri cellulaire activé par fluorescence en trois couleurs afin de former une population de cellules progénitrices endothéliales, dans laquelle lesdites cellules progénitrices endothéliales sont choisies dans le groupe constitué par les cellules progénitrices endothéliales CD34⁺CD133⁻KDR⁻, CD34⁺CD133⁺KDR⁺, CD34⁺CD133⁺KDR⁻ et CD34⁺CD133⁻ KDR⁺, l'utilisation d'embryons humains étant exclue pour des processus commerciaux ou industriels.

2. Procédé selon la revendication 1, dans lequel ladite source de cellules progénitrices est une source mammifère.

3. Procédé selon la revendication 2, dans lequel ladite source mammifère est une source humaine.

4. Procédé selon la revendication 1, dans lequel ladite source de cellules progénitrices est le sang périphérique.

5. Procédé selon la revendication 1, dans lequel lesdites cellules progénitrices endothéliales sont des cellules vasculogènes, des cellules médiatrices d'inflammation, ou les deux.

6. Procédé selon la revendication 5, dans lequel lesdites cellules vasculogènes sont des cellules progénitrices endothéliales CD34⁺CD133⁻KDR⁻.

7. Procédé selon la revendication 5, dans lequel lesdites cellules médiatrices d'inflammation sont des cellules progénitrices endothéliales CD34⁺CD133⁻KDR⁻.

8. Procédé selon la revendication 5, dans lequel lesdites cellules médiatrices d'inflammation expriment de l'interleukine 8.

9. Procédé selon la revendication 1, dans lequel lesdites cellules progénitrices endothéliales induisent des réponses angiogènes dans les vaisseaux sanguins voisins.

10. Composition thérapeutique destinée à induire une angiogenèse au niveau d'un site de traitement, comprenant :
une matrice biocompatible biodégradable dans laquelle sont disposées des cellules progénitrices endothéliales CD34⁺ négatives pour la lignée, lesdites cellules négatives pour la lignée n'exprimant pas les marqueurs de surface cellulaire CD3, CD14, CD16/56, CD19 et CD31 et lesdites cellules progénitrices endothéliales CD34⁺ étant choisies dans le groupe constitué par les cellules progénitrices endothéliales CD34⁺CD133⁻KDR⁻, CD34⁺CD133⁺KDR⁺, CD34⁺CD133⁺KDR⁻ et CD34⁺CD133⁻KDR⁺, à la condition que les cellules progénitrices ne proviennent pas d'embryons humains.

11. Composition thérapeutique selon la revendication 10, dans laquelle lesdites cellules progénitrices endothéliales CD34⁺ sont des cellules progénitrices endothéliales CD34⁺CD133⁻KDR⁻.

12. Composition thérapeutique selon la revendication 10, dans laquelle ladite matrice biocompatible biodégradable est choisie dans le groupe constitué par une membrane basale solubilisée, un gel plaquettaire autologue, des gels de collagène ou des substrats collagéniques à base d'élastine, de fibronectine, de laminine, de matrice extracellulaire et de protéines fibrillaires.

13. Composition thérapeutique ayant un effet chimiotactique sur des cellules médiatrices d'inflammation au niveau d'un site de traitement, comprenant :
une matrice biocompatible biodégradable dans laquelle sont disposées des cellules progénitrices endothéliales CD34⁺ négatives pour la lignée, lesdites cellules négatives pour la lignée n'exprimant pas les marqueurs de surface cellulaire CD3, CD14, CD 16/56, CD19 et CD31 et lesdites cellules progénitrices endothéliales CD34⁺ étant choisies dans le groupe constitué par les cellules progénitrices endothéliales CD34⁺CD133⁻KDR⁻, CD34⁺CD133⁺KDR⁺, CD34⁺CD133⁺KDR⁻ et CD34⁺CD133⁻KDR⁺, à la condition que les cellules progénitrices ne proviennent pas d'embryons humains.

14. Composition thérapeutique selon la revendication 13, dans laquelle lesdites cellules progénitrices endothéliales CD34⁺ sont des cellules progénitrices endothéliales CD34⁺CD133⁻KDR⁻.

15. Composition thérapeutique selon la revendication 13, dans laquelle ladite matrice biocompatible biodégradable est choisie dans le groupe constitué par une membrane basale solubilisée, un gel plaquettaire autologue, des gels de collagène ou des substrats collagéniques à base d'élastine, de fibronectine, de laminine, de matrice extracellulaire et de protéines fibrillaires.

16. Population isolée de cellules progénitrices endothéliales, ladite population isolée étant négative pour la lignée et CD34⁺, lesdites cellules négatives pour la lignée n'exprimant pas les marqueurs de surface cellulaire CD3, CD14, CD16/56, CD 19 et CD31 et lesdites cellules progénitrices endothéliales CD34⁺ étant choisies dans le groupe constitué par les cellules progénitrices endothéliales CD34⁺CD133⁻ KDR⁻, CD34⁺CD133⁺KDR⁺, CD34⁺CD133⁺KDR⁻ et CD34⁺CD133⁻KDR⁺, à la condition que les cellules progénitrices ne proviennent pas d'embryons humains.

17. Population isolée de cellules progénitrices endothéliales selon la revendication 16, dans laquelle ladite population isolée est négative pour la lignée et CD34⁺CD133⁻KDR⁻.

18. Population isolée de cellules progénitrices endothéliales selon la revendication 16, dans laquelle ladite population isolée comprend des cellules vasculogènes.

19. Population isolée de cellules progénitrices endothéliales selon la revendication 16, dans laquelle ladite population isolée comprend des cellules médiatrices d'inflammation.

20. Population isolée de cellules progénitrices endothéliales selon la revendication 16, dans laquelle ladite population isolée induit des réponses angiogènes dans des vaisseaux sanguins voisins.
